(19) Europäisches Patentamt
European Patent Office
Office européen des brevets

(11) Veröffentlichungsnummer: **0 093 925**
**A1**

(12) # EUROPÄISCHE PATENTANMELDUNG

(21) Anmeldenummer: **83103983.9**

(22) Anmeldetag: **23.04.83**

(51) Int. Cl.³: **C 07 D 233/58**, C 07 D 233/56, C 07 D 249/08, A 01 N 43/64, A 01 N 43/50 // C07C33/48

(30) Priorität: **06.05.82 DE 3217018**

(43) Veröffentlichungstag der Anmeldung: **16.11.83**
**Patentblatt 83/46**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Jäger, Gerhard, Dr., Gellertstrasse 18, D-5090 Leverkusen (DE)**
Erfinder: **Büchel, Karl Heinz, Prof.Dr., Dabringhausener Strasse 42, D-5093 Burscheid (DE)**
Erfinder: **Frohberger, Paul-Ernst, Dr., Willi-Baumeister-Strasse 5, D-5090 Leverkusen (DE)**
Erfinder: **Lürssen, Klaus, Dr., August-Kierspel-Strasse 89, D-5060 Bergisch-Gladbach 2 (DE)**

(54) **Azolyl-butine, Verfahren zu ihrer Herstellung sowie ihre Verwendung als Fungizide und Pflanzenwachstumsregulatoren.**

(57) Die Erfindung betrifft neue Azolyl-butin-Derivate, ein Verfahren zu ihrer Herstellung sowie ihre Verwendung als Fungizide und Pflanzenwachstumsregulatoren.

Die Verbindungen der allgemeinen Formel

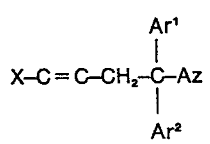

(I)

die die in der Beschreibung angegebene Bedeutung besitzen,
werden erhalten, wenn man z.B. Carbinole mit Azoliden mehrbasischer anorganischer Säuren umsetzt und gegebenenfalls anschließend die so erhaltenen Azolyl-butin-Derivate noch mit Alkalihypohalogenid umsetzt.

Die Wirkstoffe sind für den Gebrauch als Pflanzenschutzmittel geeignet und können mit besonders gutem Erfolg zur Bekämpfung von Getreidekrankheiten, von Reiskrankheiten, von Sphaerotheca-Arten und von Phytophora-Arten eingesetzt werden. Die Verbindungen besitzen außerdem das Pflanzenwachstum regulierende Eigenschaften.

EP 0 093 925 A1

BAYER AKTIENGESELLSCHAFT 5090 Leverkusen, Bayerwerk

Zentralbereich
Patente, Marken und Lizenzen Slr/AB 05.05.82
Ia

# Azolyl-butine, Verfahren zu ihrer Herstellung sowie ihre Verwendung als Fungizide und Pflanzenwachstumsregulatoren.

Die vorliegende Erfindung betrifft neue Azolyl-butin-Derivate, ein Verfahren zu ihrer Herstellung sowie ihre Verwendung als Fungizide und Pflanzenwachstumsregulatoren.

Es ist bereits bekannt geworden, daß N-Trityl-azole, wie beispielsweise 1-Triphenylmethyl-imidazol bzw. 1-Triphenylmethyl-1,2,4-triazol gute fungizide Eigenschaften aufweisen (vergleiche US-PS 3 321 366 und DE-PS 1670 976 bzw. DE-PS 1795 249). Deren Wirkung ist jedoch nicht in allen Indikationsbereichen, insbesondere bei niedrigen Aufwandmengen und -konzentrationen, immer voll befriedigend.

Es wurden neue Azolyl-butin-Derivate der allgemeinen Formel

$$X - C \equiv C - CH_2 - \overset{\displaystyle Ar^1}{\underset{\displaystyle Ar^2}{\overset{|}{\underset{|}{C}}}} - Az \qquad (I)$$

in welcher

$Ar^1$ für gegebenenfalls substituiertes Phenyl steht,

$Ar^2$ für gegebenenfalls substituiertes Phenyl steht,

Az für Imidazol-1-yl, 1,2,4-Triazol-1-yl oder 1,2,4-Triazol-4-yl steht und

X für Wasserstoff oder Halogen steht,

sowie deren Säureadditions-Salze und Metallsalz-Komplexe gefunden.

Weiterhin wurde gefunden, daß man die Azolyl-butin-Derivate der Formel (I) erhält, wenn man Carbinole der Formel

$$H - C \equiv C - CH_2 - \underset{\displaystyle Ar^2}{\overset{\displaystyle Ar^1}{\overset{|}{\underset{|}{C}}}} - OH \qquad (II)$$

in welcher

$Ar^1$ und $Ar^2$ die oben angegebene Bedeutung haben,

mit Azoliden mehrbasischer anorganischer Säuren der Formel

$$O = Y\ (Az)_n \qquad (III)$$

in welcher

Az die oben angegebene Bedeutung hat und

Y für Schwefel oder Kohlenstoff und

n für 2 steht, oder

Y für Phosphor und

n für 3 steht,

in Gegenwart eines Verdünnungsmittels umsetzt; und gegebenenfalls anschließend die so erhaltenen Azolyl-butin-Derivate der Formel

$$H-C\equiv C-CH_2-\overset{\displaystyle Ar^1}{\underset{\displaystyle Ar^2}{C}}-Az \qquad (Ia)$$

in welcher

$Ar^1$, $Ar^2$ und Az die oben angegebene Bedeutung haben,

in an sich bekannter Art und Weise mit Alkalihypohalogenit umsetzt.

An die so erhaltenen Verbindungen der Formel (I) kann gegebenenfalls eine Säure oder ein Metallsalz addiert werden.

Schließlich wurde gefunden, daß die neuen Azolyl-butin-Derivate der Formel (I) sowie deren Säureadditions-Salze und Metallsalz-Komplexe starke fungizide und pflanzenwachstumsregulierende Eigenschaften aufweisen.

Ueberraschenderweise zeigen die erfindungsgemäßen Verbindungen der Formel (I) eine bessere fungizide Wirksamkeit als die aus dem Stand der Technik bekannten N-Trityl-azole, wie beispielsweise 1-Triphenylmethyl-imidazol und 1-Triphenylmethyl-1,2,4-triazol, welche chemisch und wirkungsmäßig naheliegende Verbindungen sind. Zusätzlich zeigen die erfindungsgemäßen Verbindungen der Formel (I) überraschend gute pflanzenwachstumsregulierende Wirkung. Die erfindungsgemäßen Wirkstoffe stellen somit eine Bereicherung der Technik dar.

Die erfindungsgemäßen Azolyl-butin-Derivate sind durch die Formel (I) allgemein definiert. In dieser Formel stehen vorzugsweise

$Ar^1$ für gegebenenfalls einfach bis zweifach, gleich oder verschieden substituiertes Phenyl, wobei als Substituenten vorzugsweise genannt seien: Halogen, Alkyl mit 1 bis 4 Kohlenstoffatomen, Alkoxy mit 1 bis 4 Kohlenstoffatomen, Halogenalkyl und Halogenalkoxy mit jeweils 1 bis 2 Kohlenstoffatomen und 1 bis 5 gleichen oder verschiedenen Halogenatomen, wie Fluor- und Chloratomen, sowie gegebenenfalls durch Halogen substituiertes Phenyl;

$Ar^2$ für gegebenenfalls einfach bis zweifach, gleich oder verschieden substituiertes Phenyl, wobei als Substituenten vorzugsweise die bei $Ar^1$ bereits vorzugsweise genannten Phenylsubstituenten infrage kommen;

Az für Imidazol-1-yl, 1,2,4-Triazol-1-yl oder 1,2,4-Triazol-4-yl steht und

X für Wasserstoff, Iod, Brom oder Chlor steht.

0093925

Besonders bevorzugt sind diejenigen Verbindungen der Formel (I), in denen

$Ar^1$ für gegebenenfalls einfach bis zweifach, gleich oder verschieden substituiertes Phenyl steht, wobei als Substituenten insbesondere genannt seien: Fluor, Chlor, Brom, Methyl, Trifluormethyl, Trifluormethoxy, Phenyl und Chlorphenyl;

$Ar^2$ für gegebenenfalls einfach bis zweifach, gleich oder verschieden substituiertes Phenyl steht, wobei als Substituenten insbesondere die bei $Ar^1$ bereits insbesondere genannten Phenylsubstituenten infrage kommen;

Az für Imidazol-1-yl, 1,2,4-Triazol-1-yl oder 1,2,4-Triazol-4-yl steht und

X für Wasserstoff, Iod oder Brom steht.

Bevorzugte erfindungsgemäße Verbindungen sind auch Additionsprodukte aus Säuren und denjenigen Azolyl-butin-Derivaten der Formel (I), in denen die Substituenten $Ar^1$, $Ar^2$, Az und X die Bedeutungen haben, die bereits vorzugsweise für diese Reste genannt wurden.

Zu den Säuren die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Salizylsäure, Sorbinsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Azolyl-butin-Derivaten der Formel (I), in denen die Substituenten $Ar^1$, $Ar^2$, Az und X die Bedeutungen haben, die bereits vorzugsweise für diese Reste genannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man beispielsweise 4-(4-Chlorphenyl)-4-phenyl-1-butin-4-ol und Thionyl-bis-imidazol als Ausgangsstoffe, so kann der Reaktionsablauf durch das folgende Formelschema wiedergegeben werden:

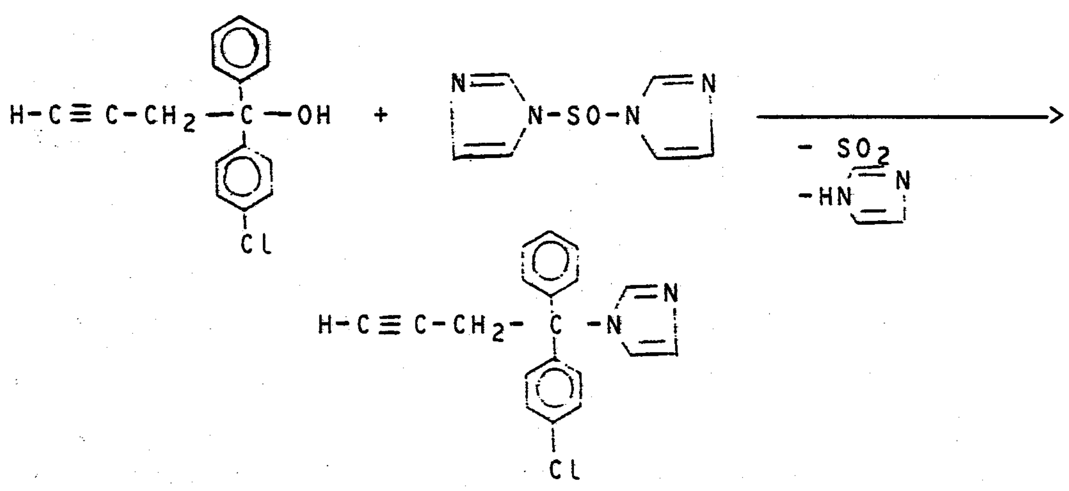

Verwendet man beispielsweise 4,4-Diphenyl-4-(1,2,4-triazol-1-yl)-1-butin und Kaliumhypobromit als Ausgangsstoffe, so kann der Reaktionsablauf durch das folgende Formelschema wiedergegeben werden:

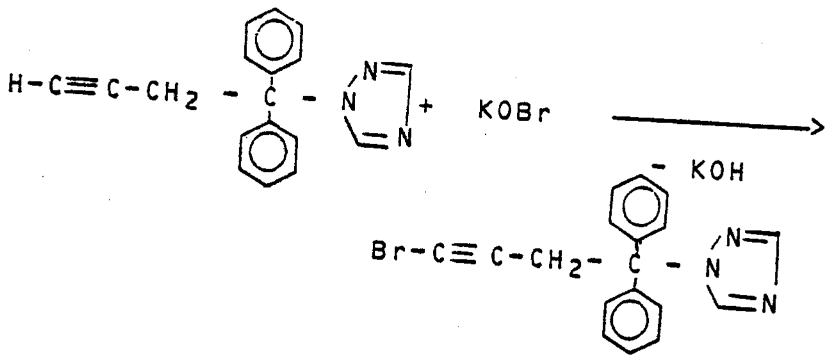


Die bei der Durchführung des erfindungsgemäßen Verfahrens als Ausgangsstoffe benötigten Carbinole sind durch die Formel (II) allgemein definiert. In dieser Formel stehen $Ar^1$ und $Ar^2$ vorzugsweise für diejenigen Bedeutungen, die bereits im Zusammenhang mit der Beschreibung der erfindungsgemäßen Stoffe der Formel (I) vorzugsweise für diese Reste genannt wurden.

Die Carbinole der Formel (II) sind bekannt, bzw. können sie in bekannter Art und Weise erhalten werden, indem man z.B. die entsprechenden Ketone in Gegenwart von aktiviertem Aluminium in einem aprotischen Lösungsmittel, wie beispielsweise Tetrahydrofuran, mit Propargylhalogeniden bei Temperaturen zwischen -70 und 60°C umsetzt (vergleiche DE-PS 1217 368; J.Agr. Food Chem. 18, 78 (1970) und Liebigs Ann.Chem. 682, 62 (1965)).

Die außerdem für das erfindungsgemäße Verfahren als Ausgangsstoffe benötigten Azolide anorganischer Säuren sind durch die Formel (III) allgemein definiert. In dieser Formel steht Az vorzugsweise für diejenigen Bedeutungen, die bereits im Zusammenhang mit der Beschreibung der erfindungsgemäßen Stoffe der Formel (I) vorzugsweise für diesen Rest genannt wurden. Y und der Index n stehen vorzugsweise für die in der Erfindungsdefinition angegebenen Bedeutungen.

Die Azolide der Formel (III) sind allgemein bekannte Verbindungen der organischen Chemie. Sie werden vorzugsweise in situ erzeugt, indem man z.B. Thionylchlorid bei Raumtemperatur zu einer Lösung von N-Trimethylsilylimidazol in absolutem Toluol gibt.

Für die erfindungsgemäße Umsetzung der Carbinole der Formel (II) mit den Azoliden der Formel (III) kommen als Verdünnungsmittel vorzugsweise polare organische Lösungsmittel infrage. Hierzu gehören beispielsweise Nitrile, wie Acetonitril; Sulfoxide, wie Dimethylsulfoxid; Formamide, wie Dimethylformamid; Ketone, wie Aceton; Ether, wie Diethylether und Tetrahydrofuran; sowie Chlorkohlenwasserstoffe, wie Methylenchlorid und Chloroform.

Die Reaktionstemperaturen können bei dieser erfindungsgemäßen Umsetzung in einem größeren Bereich variiert werden. Im allgemeinen arbeitet man zwischen -60°C und 100°C, vorzugsweise zwischen -50 und 85°C.

Bei der Durchführung dieser erfindungsgemäßen Umsetzung setzt man auf 1 Mol der Carbinole der Formel (II) vorzugsweise 2 bis 3 Mol des Azolids der Formel (III) ein, wobei das Azolid der Formel (III) vorzugsweise in situ erzeugt wird. Die Isolierung der Verbindungen der Formel (I) erfolgt in üblicher Weise.

Für die erfindungsgemäße Umsetzung der Azolyl-butin-Derivate der Formel (Ia) mit Alkalihypohalogenit kommen als Verdünnungsmittel Wasser sowie gegenüber Alkalihypohalogenit inerte organische Lösungsmittel infrage. Hierzu gehören beispielsweise Alkohle, wie Methanol oder Ethanol; Ether, wie Diethylether, Dioxan oder Tetrahydrofuran; sowie auch Zweiphasengemische, wie z.B. Ether/Wasser.

Die Reaktionstemperaturen können bei dieser erfindungsgemäßen Umsetzung in einem größeren Bereich variiert werden. Im allgemeinen arbeitet man zwischen 0 und 60°C, vorzugsweise zwischen 0 und 40°C.

Bei der Durchführung dieser erfindungsgemäßen Umsetzung setzt man auf 1 Mol der Verbindung der Formel (Ia) vorzugsweise 1 bis 1,5 Mol Hypohalogenit ein, wobei das Alkalihypohalogenit in situ aus dem entsprechenden Halogen und dem Alkalihydroxid, insbesondere Natrium- und Kaliumhydroxid, erzeugt wird (vergleiche hierzu auch Houben-Weyl, Band V/2a, Seiten 608-10 (1977)). Die Isolierung der Endprodukte erfolgt in üblicher Weise.

Zur Herstellung von Säureadditions-Salzen der Verbindungen der Formel (I) kommen vorzugsweise diejenigen Säuren infrage, die bereits im Zusammenhang mit der Beschreibung der erfindungsgemäßen Säureadditions-Salze als bevorzugte Säuren genannt wurden.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I.bis II. sowie IV.bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindungen der Formel(I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie z.B. gegen den Erreger des Gerstenmehltaus (Erysiphe graminis), von Reiskrankheiten, wie z.B. Pyricularia oryzae, von Sphaerotheca-Arten, wie z.B. den Erreger des Gurkenmehltaus (Spaerotheca fuliginea) sowie von Phytophora Arten, wie z.B. gegen den Erreger der Braunfäule der Tomaten (Phytophthora infestans) eingesetzt werden.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung aufgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z.B. bei Tabak,

Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und / oder festen Trägerstoffen, gegebenenfalls unter Verwendung von oberflächenaktiven Mitteln, also Emulgiermitteln und / oder Dispergiermitteln und / oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und / oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie

Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen o,1 und 95 Gewichtsprozent Wirkstoff, vorzugsweise zwischen o,5 und 9o %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach klimatischen und vegetativen Gegebenheiten richtet.

Auch beim Einsatz der erfindungsgemäßen Stoffe als Fungizide kann die Aufwandmenge je nach Art der Applikation in einem größeren Bereich variiert werden. So liegen die Wirkungskonzentrationen bei der Behandlung von Pflanzenteilen in den Aufwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50 g je kg Saatgut, vorzugsweise 0,01 bis 10g, benötigt. Bei der Behandlung des Bodens sind Wirkstoffkonzentrationen von 0,00001 bis 0,1 Gew.-%, vorzugsweise von 0,0001 bis 0,02 %, am Wirkungsort erforderlich.

0093925

Herstellungsbeispiele

Beispiel 1

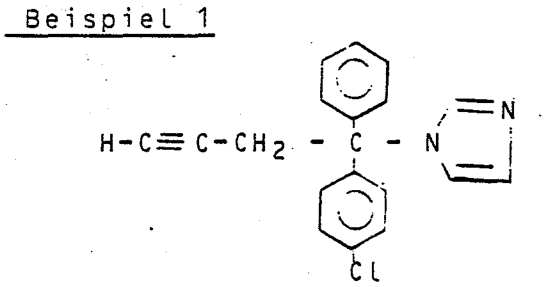

Zu einer Lösung von 224g (1,6 Mol) N-Trimethylsilylimidazol in 1500 ml absolutem Toluol werden bei Raumtemperatur 95,2 g (0,8 Mol) Thionylchlorid innerhalb einer halben Stunde eingerührt. Man dekantiert das Toluol vom ausgeschiedenen Thionyl-bis-imidazol ab, nimmt in 800 ml Acetonitril auf, kühlt die Lösung auf -40°C und tropft bei dieser Temperatur eine Lösung von 102,6g (0,4 Mol) 4-(4-Chlorphenyl)-4-phenyl-1-butin-4-ol unter Rühren zu. Das Reaktionsgemisch wird noch 1 Stunde bei -40°C und anschließend 3 Stunden bei 0°C gehalten. Man destilliert das Lösungsmittel unter vermindertem Druck ab, nimmt den Rückstand in 300 ml Essigester auf, wäscht dreimal mit je 100 ml Wasser und dampft die organische Phase ein. Der verbleibende flüssige Rückstand (124,1 g) wird in 300 ml Aceton gelöst und langsam mit 43,2g (0,15 Mol) Naphthalin-1,5-disulfonsäure, gelöst in 150 ml Aceton, versetzt. Nach einstündigem Stehen wird das auskristallisierte Salz abfiltriert und mit Aceton gewaschen. (Ausbeute: 54,7 g, 23 % der Theorie; Schmp. 209-211°C). Zur Freisetzung der Base wird das Naphthalin-1,5-disulfonat zwischen je 150 ml Wasser und Dichlormethan verteilt und mit 100 ml wäßriger Natriumcarbonat-Lösung versetzt. Man trennt die organische Phase ab, trocknet über wasserfreiem Natriumsulfat und destilliert das Lösungsmittel unter vermindertem Druck ab. Es werden so 27,6 g (22,5 % der Theorie) 4-(4-Chlorphenyl)-4-(1-imidazolyl)-4-phenyl-1-butin in Form farbloser Kristalle vom Schmelzpunkt 117-118°C erhalten.

Herstellung des Ausgangsproduktes

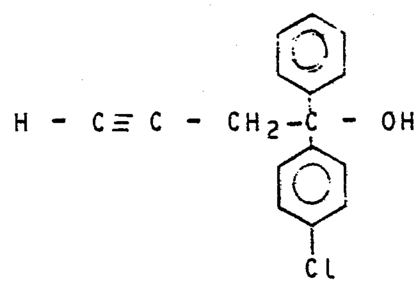

52g Aluminiumspäne werden mit 240 ml trockenem Tetrahydrofuran überschichtet. Nach Zugabe von je einer Spatelspitze Iod und Quecksilber-(II)-chlorid wird das Gemisch bei Raumtemperatur zwölf Stunden gerührt. Anschließend wird auf 60°C erwärmt und eine Lösung von 338g (2,84 Mol) Propargylbromid in 340 ml Tetrahydrofuran innerhalb 1 Stunde unter Rühren eingetropft.

Man kühlt das Reaktionsgemisch auf -60°C ab und tropft innerhalb von 1,5 Stunden 432g (2 Mol) 4-Chlorbenzophenon, gelöst in 660 ml Tetrahydrofuran, zu. Anschließend läßt man auf 0°C erwärmen, versetzt mit 680 ml einer gesättigten wäßrigen Ammoniumchlorid-Lösung, filtriert und destilliert die Hauptmenge des Tetrahydrofurans unter vermindertem Druck ab. Der Rückstand wird mit 1000 ml Essigester versetzt und das Gemisch viermal mit je 700 ml Wasser gewaschen. Die organische Phase wird über Natriumsulfat getrocknet, das Lösungsmittel im Wasserstrahl-Vakuum abdestilliert und der verbleibende ölige Rückstand mit Petrolether versetzt. Man erhält so 366,8g (71,6 % der Theorie) 4-(4-Chlorphenyl)-4-phenyl-1-butin-4-ol vom Schelzpunkt 58-59°C.

Beispiel 2 und 3

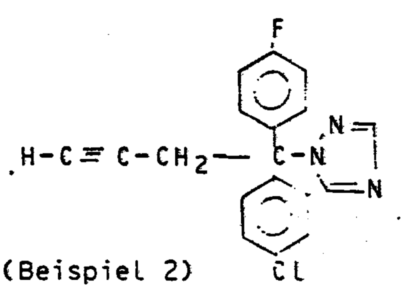

(Beispiel 2)

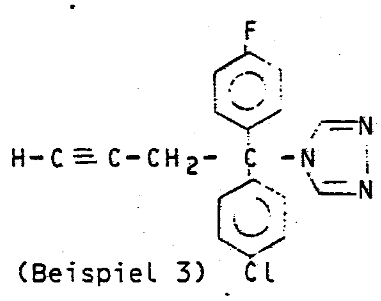

(Beispiel 3)

Unter Rühren werden bei 5 bis 10°C 47,6 g (0,4 Mol) Thionylchlorid zu einer Lösung von 112,8g (0,8 Mol) N-Trimethylsilyltriazol in 1000ml trockenem Toluol zugetropft. Man filtriert das ausgeschiedene Thionyl-bis-triazol ab, suspendiert es in 400 ml Acetonitril und rührt in diese Suspension bei -40°C eine Lösung von 55g (0,2 Mol) 4-(4-Chlorphenyl)-4-(4-fluorphenyl)-1-butin-4-ol in 80ml Acetonitril innerhalb von 30 Minuten ein. Man rührt noch eine Stunde bei -40°C und 3 Stunden bei 0°C nach und engt anschließend das Reaktionsgemisch unter vermindertem Druck ein. Der Rückstand wird in 300 ml Essigester aufgenommen und dreimal mit je 50ml Wasser gewaschen. Nach dem Eindampfen der organischen Phase erhält man 63g einer viskosen Flüssigkeit. Durch säulenchromatographische Trennung an Kieselgel (Eluierungsmittel: a) Cyclohexan/ Chloroform 3:1; b) Chloroform) erhält man 9,5g (14,6 % der Theorie) 4-(4-Chlorphenyl)-4-(4-fluorphenyl)-4-(1,2,4-triazol-1-yl)-1-butin (Beispiel 2) vom Schmelzpunkt 84-85°C und 13,9g (21,3 % der Theorie) 4-(4-Chlorphenyl)-4-(4-fluorphenyl)-4-(1,2,4-triazol-4-yl)-1-butin vom Schmelzpunkt 139-140°C (Beispiel 3).

Beispiel 4

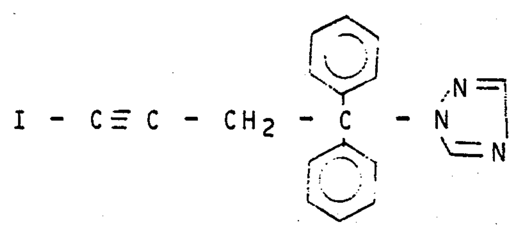

Zu einer Lösung von 13,7g (0,05 Mol) 4,4-Diphenyl-4-(1,2,4-triazol-1-yl)-1-butin in 150 ml Methanol werden bei 20 bis 25°C unter gleichzeitigem Zutropfen von 24 ml konzentrierter Natronlauge 15,3 g(0,06 Mol) Iod eingetragen. Nach vier Stunden werden die ausgeschiedenen Kristalle abfiltriert, mit Wasser und Methanol gewaschen und im Vakuum getrocknet. Man erhält so 12,0g (60 % der Theorie) 4,4-Diphenyl-1-iod-4-(1,2,4-triazol-1-yl)-1-butin vom Schmelzpunkt 158-160°C.

0093925

In entsprechender Weise und gemäß den angegebenen erfindungsgemäßen Umsetzungen werden die folgenden Verbindungen der allgemeinen Formel

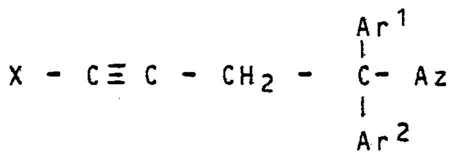 (I)

erhalten:

| Bsp. Nr. | $Ar^1$ | $Ar^2$ | Az | X | Schmelzpunkt(°C) |
|---|---|---|---|---|---|
| 5 | 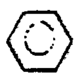 | 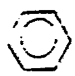 | 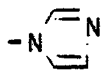 | H | 93-95 |
| 6 | 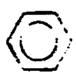 | 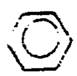 | 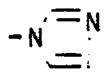 | H | 232(Zers.) (x 1/2 NDS) |
| 7 | 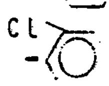 | 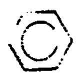 | 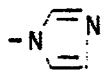 | H | 222(Zers.) (x 1/2 NDS) |
| 8 | 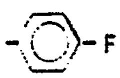 | 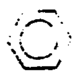 | 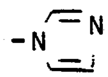 | H | 90-92 |
| 9 | 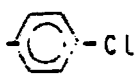 | 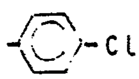 | 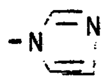 | H | 133-36 |
| 10 | 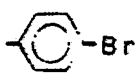 | 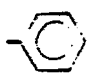 | 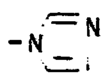 | H | 111-12 |
| 11 | 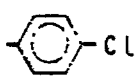 | 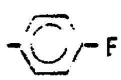 | 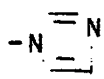 | H | 116-17 |
| 12 | 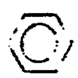 | 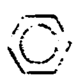 | 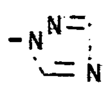 | H | 105-06 |
| 13 | 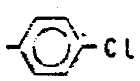 | 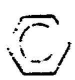 | 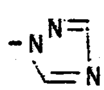 | H | 138-39 |
| 14 | 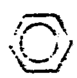 | 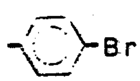 | 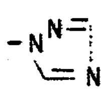 | H | 156-58 |
| 15 | 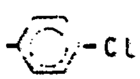 | 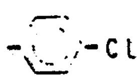 | 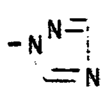 | H | 114-15 |

0093925

| Bsp. Nr. | $Ar^1$ | $Ar^2$ | Az | X | Schmelz- punkt(°C) |
|---|---|---|---|---|---|
| 16 | 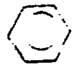 | 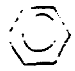 | 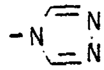 | H | 125-26 |
| 17 | 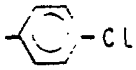 | 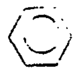 | 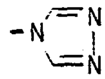 | H | 118-19 |
| 18 | 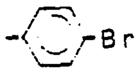 | 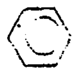 | 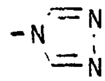 | H | 108-09 |
| 19 | 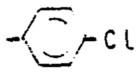 | 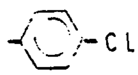 | 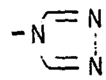 | H | 188-89 |
| 20 | 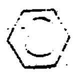 | 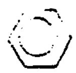 | 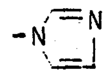 | I | 173-74 |
| 21 | 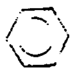 | 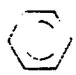 | 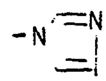 | Br | 114-15 |
| 22 | 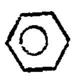 | 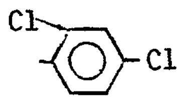 | 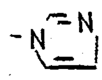 | H | 150(x 1/2 NDS) |

NDS = 1,5-Naphthalindisulfonsäure

Anwendungsbeispiele

In dem nachfolgenden fungiziden Anwendungsbeispiel werden die nachstehend angegebenen Verbindungen als Vergleichssubstanzen eingesetzt:

(A) 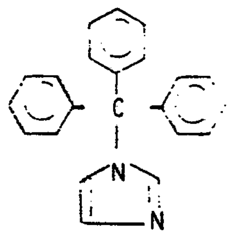

(B) 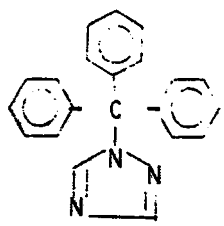

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20° C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 5,6,20,21,12,4,8,1,9,10 und 11.

Beispiel B

Stimulierung der $CO_2$-Fixierung bei Sojabohnen

Lösungsmittel:30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Im weiteren Verlauf des Experimentes wird die $CO_2$-Fixierung der Pflanzen mit üblichen Methoden gemessen. Die Werte werden mit denen nicht mit Wirkstoffen behandelter Kontrollpflanzen vergleichen.

Weitere Versuchsdaten und die Resultate dieses Versuches gehen aus der nachfolgenden Tabelle hervor.

Der erfindungsgemäße Wirkstoff gemäß Herstellungsbeispiel 14 zeigt in diesem Test im Vergleich zur Kontrolle eine sehr starke Stimulierung der $CO_2$-Fixierung.

Patentansprüche

1. Azolyl-butin-Derivate der allgemeinen Formel

$$X - C \equiv C - CH_2 - \overset{Ar^1}{\underset{Ar^2}{C}} - Az \qquad (I)$$

in welcher

$Ar^1$ für gegebenenfalls substituiertes Phenyl steht,

$Ar^2$ für gegebenenfalls substituiertes Phenyl steht,

Az für Imidazol-1-yl, 1,2,4-Triazol-1-yl oder 1,2,4-Triazol-4-yl steht und

X für Wasserstoff oder Halogen steht,

sowie deren Säureadditions-Salze und Metallsalz-Komplexe.

2. Verbindungen der allgemeinen Formel I in Anspruch 1, wobei

$Ar^1$ für gegebenenfalls einfach bis zweifach, gleich oder verschieden durch Fluor, Chlor, Brom, Methyl, Trifluormethyl, Trifluormethoxy, Phenyl und Chlorphenyl substituiertes Phenyl steht,

$Ar^2$ für gegebenenfalls einfach bis zweifach, gleich oder verschieden durch die bei $Ar^1$ bereits genannten Phenylsubstituenten sub-

stituiertes Phenyl steht,

Az für Imidazol — 1-yl, 1,2,4-Triazol-1-yl oder 1,2,4-Triazol-4-yl steht und

X für Wasserstoff, Iod oder Brom steht.

3. Verfahren zur Herstellung von Azolyl-butin-Derivaten der allgemeinen Formel

$$X - C\equiv C - CH_2 - \overset{\displaystyle Ar^1}{\underset{\displaystyle Ar^2}{C}} - Az \qquad (I)$$

in welcher

$Ar^1$ für gegebenenfalls substituiertes Phenyl steht,

$Ar^2$ für gegebenenfalls substituiertes Phenyl steht,

Az für Imidazol-1-yl, 1,2,4-Triazol-1-yl oder 1,2,4-Triazol-4-yl steht und

X für Wasserstoff oder Halogen steht,

dadurch gekennzeichnet, daß man Carbinole der Formel

$$H - C\equiv C - CH_2 - \overset{\displaystyle Ar^1}{\underset{\displaystyle Ar^2}{C}} - OH \qquad (II)$$

in welcher

$Ar^1$ und $Ar^2$ die oben angegebene Bedeutung haben,

mit Azoliden mehrbasischer anorganischer Säuren der Formel

$$O = Y\,(Az)_n \qquad (III)$$

in welcher

Az die oben angegebene Bedeutung hat und

Y für Schwefel oder Kohlenstoff und

n für 2 steht, oder

Y für Phosphor und

n für 3 steht,

in Gegenwart eines Verdünnungsmittels umsetzt; und gegebenenfalls anschließend die so erhaltenen Azolylbutin-Derivate der Formel

$$H - C\equiv C - CH_2 - \underset{Ar^2}{\overset{Ar^1}{C}} - Az \qquad (Ia)$$

in welcher

$Ar^1$, $Ar^2$ und Az die oben angegebene Bedeutung haben,

in an sich bekannter Art und Weise mit Alkalihypohalogenit umsetzt, und an die so erhaltenen Verbindungen der Formel (I) gegebenenfalls noch eine Säure

oder ein Metallsalz addiert.

4. Pflanzenschutzmittel, gekennzeichnet durch einen Gehalt an mindestens einem Azolyl-butin-Derivat der Formel I in Ansprüchen 1 und 3.

5. Fungizide Mittel und das Wachstum von Pflanzen regulierende Mittel, gekennzeichnet durch einen Gehalt an mindestens einem Azolyl-butin-Derivat der Formel I in Ansprüchen 1 und 3.

6. Verfahren zur Bekämpfung von Pilzen und zur Regulierung des Pflanzenwachstums, dadurch gekennzeichnet, daß man Azolyl-butin-Derivate der Formel 1 in Ansprüchen 1 und 3 auf Pilze, zu behandelnde Pflanzen oder ihren Lebensraum einwirken läßt.

7. Verwendung von Azolyl-butin-Derivaten der Formel I in Ansprüchen 1 und 3 als Pflanzenschutzmittel.

8. Verwendung von Azolyl-butin-Derivaten der Formel I in Ansprüchen 1 und 3 zur Bekämpfung von Pilzen.

9. Verwendung von Azolyl-butin-Derivaten der Formel I in Ansprüchen 1 und 3 zur Regulierung des Pflanzenwachstums.

10. Verfahren zur Herstellung von Pflanzenschutzmitteln, dadurch gekennzeichnet, daß man Azolyl-butin-Derivate der Formel I in Ansprüchen 1 und 3 mit Streckmitteln und/oder oberflächenaktiven Mitteln vermischt.

Europäisches Patentamt

**EUROPÄISCHER RECHERCHENBERICHT**

0093925

Nummer der Anmeldung

EP 83 10 3983

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A,P | EP-A-0 052 424 (ICI)<br>* Ansprüche *<br>--- | 1,5,6,8 | C 07 D 233/58<br>C 07 D 233/56<br>C 07 D 249/08<br>A 01 N 43/64<br>A 01 N 43/50 //<br>C 07 C 33/48 |
| A,P | GB-A-1 332 393 (BAYER)<br>* Ansprüche *<br>----- | 1 | |
| | | | **RECHERCHIERTE SACHGEBIETE (Int. Cl. 3)** |
| | | | C 07 D 233/00<br>C 07 D 249/00 |

Der vorliegende Recherchenbericht wurde für alle Patentansprüche erstellt.

| Recherchenort | Abschlußdatum der Recherche | Prüfer |
|---|---|---|
| DEN HAAG | 19-08-1983 | CREMERS K. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument